# EUROPEAN PATENT APPLICATION

(11) **EP 4 008 315 A1**
(43) Date of publication of application: **08.06.2022**
(21) Application number: 21211881.4
(22) Date of filing: 02.12.2021
(51) Int. Cl.: A61K 9/20, A61K 9/28

(54) **A PROCESS FOR FORMULATIONS OF DAPAGLIFLOZIN AND METFORMIN HYDROCHLORIDE**

(30) Priority: 03.12.2020 TR 202019590
(71) Applicant: SANOVEL ILAC SANAYI VE TICARET A.S., Istanbul 34460 (TR)
(72) Inventor: PEHLIVAN AKALIN, Nur, 34460 Istanbul (TR); SÜNEL, Fatih, 34460 Istanbul (TR); OZDEN, Aydan, 34460 Istanbul (TR); ARSIN, Gülcan, 34460 Istanbul (TR)
(74) Representative: Sevinç, Erkan

(57) **Abstract**

The present invention relates to a process for the preparation of a film coated tablet formulation comprising dapagliflozin and metformin hydrochloride. The process is simple, rapid, cost effective, time-saving and industrially convenient process and is created to eliminate the disadvantages of both active ingredients.

## Description

### Field of the Invention

The present invention relates to a process for the preparation of a film coated tablet formulation comprising dapagliflozin and metformin hydrochloride. The process is simple, rapid, cost effective, time-saving and industrially convenient process and is created to eliminate the disadvantages of both active ingredients.

### Background of the Invention

Diabetes mellitus is a group of disorders of carbohydrate metabolism in which the action of insulin is diminished or absent through altered secretion, decreased insulin activity or a combination of both factors. There are two main types of diabetes; Type 1 and Type 2:

Type 1 diabetes occurs because the insulin-producing cells of the pancreas (beta cells) are damaged. In Type 1 diabetes, the pancreas makes little or no insulin, so sugar cannot get into the body's cells for use as energy. People with Type 1 diabetes must use insulin injections to control their blood glucose.

In Type 2 diabetes, the pancreas makes insulin, but it either doesn't produce enough, or the insulin does not work properly. This diabetes occurs most often in people who are over 40 years old and overweight and type 2 diabetes is the most common type, affecting more than 171 million people worldwide.Type 2 diabetes may sometimes be controlled with a combination of diet, weight management, and exercise. However, treatment also may include oral glucose-lowering medications or insulin injections.

Dapagliflozin is a sodium-glucose cotransporter 2 inhibitor (SGLT2). SGLT2 is a carrier responsible for the reabsorption of most of the glucose from the lumen of the renal tubule. SGLT2 is expressed in proximal renal tubules. By inhibiting SGLT2, dapagliflozin reduces the reabsorption of the filtered glucose and lowers the renal threshold for glucose. This improves urinary glucose excretion and blood glucose control. Dapagliflozin, also known as (1S)-1,5-Anhydro-1-C-[4-chloro-3-[(4-ethoxyphenyl)methyl]phenyl]-D-glucitol or (2S,3R,4R,5S,6R)-2-(3-(4-etoxybenzyl)-4-chlorophenyl)-6-hydroxymethyltetrahydro-2H-pyran-3,4,5-triol is represented by the structure of Formula I.

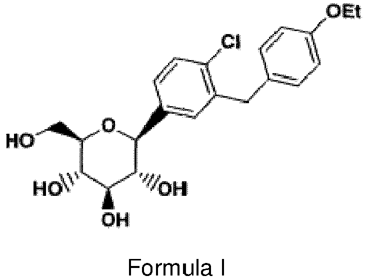

Dapagliflozin was first disclosed in patent US 6515117 (2003, Bristol-Myers Squibb).

Metformin is antidiabetics having an orally-administrated biguanide structure. Metformin hydrochloride is a white to off-white crystalline compound and it is freely soluble in water and practically insoluble in acetone, ether and chloroform. Oral doses of metformin are generally recommended in the range of 500 to 2500 mg a day and a single dose may vary from 250 to 1000mg. It is used singly or in combination with sulfonylureas, alpha-glucosidase inhibitors, or insulin.

The chemical name of metformin hydrochloride is 1,1-dimethylbiguanide hydrochloride, has the following chemical structure of Formula II.

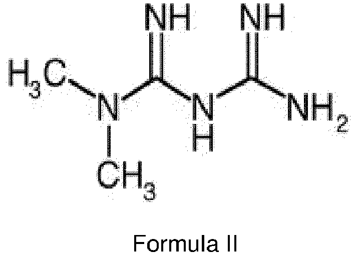

Metformin was first disclosed in patent US 3174901 (1965, Jan Marcel Didier Aron-Samuel).

Dapagliflozin and metformin, sold under the brand name Xigduo film coated tablet among others, is a fixed-dose combination anti-diabetic medication used as an adjunct to diet and exercise to improve glycemic control in adults with type 2 diabetes.

EP2498759 (A2) discloses an immediate release pharmaceutical formulation which includes a tablet or capsule formulation comprising metformin and dapagliflozin or its propylene glycol hydrate. The present invention also provides methods of preparing the formulations.

WO 2011/060256 relates to bilayer tablet formulations comprising metformin extended release (XR) or reduced mass metformin XR formulation as the first layer, an SGLT2 inhibitor (dapagliflozin) formulation as the second layer, and optionally a film coating. The formulation is prepared by direct compression method.

The dapagliflozin is hygroscopic in nature. It absorbs moisture and forms sticky lumps which are difficult to process and handle, and which may ultimately lead to content uniformity issues in the dosage form. Dapagliflozin has the low solubility and stability which may lead to poor bioavailability of the drug.

Also, as known, metformin is a very poorly compressible active substance and in the present invention, metformin presents in high amounts in the tablet.

Another problem encountered while developing combination of said active agents is the flowability and compressibility problem, which makes the production difficult.

There is thus still a need for a process for the film coated tablet comprising dapagliflozin and metformin hydrochloride that provides a tablet having the desired stability, content uniformity, flowability and compressibility, in another words the disadvantages seen in the active substances will able to overcome. In the present invention, the process is using standard techniques which is simple and cost-effective method.

### Detailed Description of the Invention

The main object of the present invention is to provide an effective process for the preparation of a film coated tablet formulation comprising dapagliflozin and metformin hydrochloride which eliminate all aforesaid problems and bring additional advantages to the relevant prior art. The process provides the desired content uniformity, flowability and compressibility of the film coated tablet.

Another object of the present invention is to obtain an effective process for the preparation of a film coated tablet formulation comprising dapagliflozin and metformin hydrochloride which has the desired dissolution and the desired stability.

Dapagliflozin is used small proportion that can lead to considerable problems during the manufacture of the composition with regard to the uniformity of the content of active agent in the individual composition units.

Also, as known, metformin is a very poorly compressible active substance and in the present invention, metformin presents in high amounts in the tablet.

The advantages of the present invention are even more significant, as the problem of homogeneity is even more likely to occur when two active substances are incorporated in one final dosage form, especially when two actives is used very different regarding the amount. Improved content uniformity efficiently contributes to a marked increase in bioavailability. Improved content uniformity also favors to avoid toxicity in the otherwise possible event that the amount of drug substance would be too high.

It has surprisingly been found that a film coated tablet of excellent content uniformity, good flowability and showing improved dissolution can be obtained when dapagliflozin and metformin hydrochloride are formulated together in wet granulation process. Wet granulation process efficiently counteracts the segregation of active agents, so it is observed that the desired stability, flowability, content uniformity and compressibility.

A process for the preparation of the film coated tablet comprising dapagliflozin and metformin hydrochloride comprising the following steps:
a) Mixing dapagliflozin, metformin hydrochloride, at least one disintegrant and at least one filler,
b) Dissolving at least binder in a solvent to obtained a solution,
c) Granulating the solution with the mixture at step (a).

So, an easy method is created to eliminate the disadvantages of both active ingredients and also the method is simple and cost-effective method. Also, this process helps to provide the desired dissolution profile.

In the process, metformin HCI and dapagliflozin are put into the tank at the same time with at least one disintegrant and at least one filler, Thus, the desired mixture can be achieved without loss of active ingredient.

In the process, when a solution is prepared with at least binder, then mixing with the mixture comprising active agents, it was observed that the desired content uniformity is provided. Especially, the problem caused by the use of small amounts of dapagliflozin has been prevented.

Suitable disintegrants are selected from the group comprising croscarmellose sodium, sodium starch glycollate, crospovidone, sodium alginate, gums, starch and magnesium aluminum silicate or a mixture thereof.

According to an embodiment of the present invention, the disintegrant is croscarmellose sodium.

Suitable fillers are selected from the group comprising microcrystalline cellulose, lactose, starch, mannitol, calcium hydrogen phosphate dihydrate, dicalcium hydrogen phosphate anhydrate, calcium phosphate trihydrate, silicon dioxide, neutral pellets, magnesium carbonate, magnesium oxide, maltodextrin, maltose, medium chain triglycerides or mixtures thereof.

According to an embodiment of the present invention, the filler is microcrystalline cellulose.

Suitable binders are selected from the group comprising polyvinylpyrrolidone, hydroxypropylmethylcellulose, lactose anhydrous, pregelatinized starch, calcium carbonate, calcium phosphate dibasic, calcium phosphate tribasic, calcium sulphate, hydroxypropylcellulose, carboxymethylcellulose, methyl cellulose, ethyl cellulose or mixtures thereof.

According to an embodiment of the present invention, the binder is polyvinylpyrrolidone.

According to an embodiment of the present invention, solvents are selected from the group comprising pure water, propylene glycol, glycerin, ethanol, polyethylene glycol or mixtures thereof.

According to an embodiment of the present invention, the solvent is pure water.

According to an embodiment of the present invention, spray granulation is used at step (c).

According to one embodiment of the present invention, the process for the preparation of the film coated tablet further comprises the following steps:
d) Drying the mixture, then sieving the mixture,
e) Adding at least one lubricant and mixing,
f) Compressing the mixture to form a tablet,
g) Coating tablets with film coating agents.

Suitable lubricants are selected from the group comprising magnesium stearate, silica colloidal anhydrous, sodium stearyl fumarate, talc, polyethylene glycol, stearic acid, aluminum silicate or mixtures thereof.

According to an embodiment of the present invention, the lubricant is magnesium stearate.

According to an embodiment of the present invention, film coating agents are polyvinyl alcohol, talc, titanium dioxide, polyethylene glycol, iron oxide.

According to one embodiment of the present invention, the process for the preparation of the film coated tablet comprises the following steps:
a) Mixing dapagliflozin, metformin hydrochloride, croscarmellose sodium and microcrystalline cellulose,
b) Dissolving polyvinylpyrrolidone in pure water to obtained a solution,
c) Granulating the solution with the mixture at step (a).
d) Drying the mixture, then sieving the mixture,
e) Adding magnesium stearate and mixing,
f) Compressing the mixture to form a tablet,
g) Coating tablets with film coating agents.

### Example 1: The film coated tablet

| **Ingredients** | **Ingredients in one tablet (%) (by weight)** |
|---|---|
| Dapagliflozin | 0.05 - 5.0 |
| Metformin HCI | 55.0 - 80.0 |
| Polyvinylpyrrolidone | 2.0 - 15.0 |
| Microcrystalline cellulose | 5.0 - 30.0 |
| Croscarmellose sodium | 1.0 - 10.0 |
| Magnesium stearate | 0.1 -8.0 |
| Film coating agents (polyvinyl alcohol, talc, titanium dioxide, polyethylene glycol, iron oxide) | 2.0-10.0 |
| **Total** | **100** |

### Example 2: The film coated tablet

| **Ingredients** | **Ingredients in one tablet (%) (by weight)** |
|---|---|
| Dapagliflozin | 0.3 |
| Metformin HCI | 68.5 |
| Polyvinylpyrrolidone | 5.5 |
| Microcrystalline cellulose | 16.8 |
| Croscarmellose sodium | 2.4 |
| Magnesium stearate | 1.0 |
| Film coating agents (polyvinyl alcohol, talc, titanium dioxide, polyethylene glycol, iron oxide) | 5.5 |
| **Total** | **100** |

A process for example 1 or 2;
a) Mixing dapagliflozin, metformin hydrochloride, croscarmellose sodium and microcrystalline cellulose,
b) Dissolving polyvinylpyrrolidone in pure water to obtained a solution,
c) Granulating the solution with the mixture at step (a).
d) Drying the mixture, then sieving the mixture,
e) Adding magnesium stearate and mixing,
f) Compressing the mixture to form a tablet,
g) Coating tablets with film coating agents.

## Claims

1. A process for the preparation of the film coated tablet comprising dapagliflozin and metformin hydrochloride comprising the following steps:
a) Mixing dapagliflozin, metformin hydrochloride, at least one disintegrant and at least one filler,
b) Dissolving at least binder in a solvent to obtained a solution,
c) Granulating the solution with the mixture at step (a).

2. The process for the preparation of the film coated tablet according to claim 1, wherein disintegrants are selected from the group comprising croscarmellose sodium, sodium starch glycollate, crospovidone, sodium alginate, gums, starch and magnesium aluminum silicate or a mixture thereof.

3. The process for the preparation of the film coated tablet according to claim 2, wherein the disintegrant is croscarmellose sodium.

4. The process for the preparation of the film coated tablet according to claim 1, wherein fillers are selected from the group comprising microcrystalline cellulose, lactose, starch, mannitol, calcium hydrogen phosphate dihydrate, dicalcium hydrogen phosphate anhydrate, calcium phosphate trihydrate, silicon dioxide, neutral pellets, magnesium carbonate, magnesium oxide, maltodextrin, maltose, medium chain triglycerides or mixtures thereof.

5. The process for the preparation of the film coated tablet according to claim 4, wherein the filler is microcrystalline cellulose.

6. The process for the preparation of the film coated tablet according to claim 1, wherein binders are selected from the group comprising polyvinylpyrrolidone, hydroxypropylmethylcellulose, lactose anhydrous, pregelatinized starch, calcium carbonate, calcium phosphate dibasic, calcium phosphate tribasic, calcium sulphate, hydroxypropylcellulose, carboxymethylcellulose, methyl cellulose, ethyl cellulose or mixtures thereof.

7. The process for the preparation of the film coated tablet according to claim 6, wherein the binder is polyvinylpyrrolidone.

8. The process for the preparation of the film coated tablet according to claim 1, wherein solvents are selected from the group comprising pure water, propylene glycol, glycerin, ethanol, polyethylene glycol or mixtures thereof.

9. The process for the preparation of the film coated tablet according to claim 9, wherein the solvent is pure water.

10. The process for the preparation of the film coated tablet according to claim 1, wherein spray granulation is used at step (c).

11. The process for the preparation of the film coated tablet according to claim 1, further comprising;
d) Drying the mixture, then sieving the mixture,
e) Adding at least one lubricant and mixing,
f) Compressing the mixture to form a tablet,
g) Coating tablets with film coating agents.

12. The process for the preparation of the film coated tablet according to claim 11, wherein lubricants are selected from the group comprising magnesium stearate, silica colloidal anhydrous, sodium stearyl fumarate, talc, polyethylene glycol, stearic acid, aluminum silicate or mixtures thereof.

13. The process for the preparation of the film coated tablet according to claim 12, wherein the lubricant is magnesium stearate.

14. The process for the preparation of the film coated tablet according to claim 11, wherein film coating agents are polyvinyl alcohol, talc, titanium dioxide, polyethylene glycol, iron oxide.

15. The process for the preparation of the film coated tablet according to claim 1, comprising;
a) Mixing dapagliflozin, metformin hydrochloride, croscarmellose sodium and microcrystalline cellulose,
b) Dissolving polyvinylpyrrolidone in pure water to obtained a solution,
c) Granulating the solution with the mixture at step (a).
d) Drying the mixture, then sieving the mixture,
e) Adding magnesium stearate and mixing,
f) Compressing the mixture to form a tablet,
g) Coating tablets with film coating agents.
